# EUROPEAN PATENT APPLICATION

(11) **EP 4 703 369 A1**
(43) Date of publication of application: **04.03.2026**
(21) Application number: 23936227.0
(22) Date of filing: 07.06.2023
(51) Int. Cl.: C07K 1/06, C07K 1/02

(54) **METHOD FOR SYNTHESIZING AMIDE AND/OR POLYPEPTIDE BY USING TEMPORARILY PROTECTED AMINO ACIDS AS AMINE COMPONENTS**

(30) Priority: 05.05.2023 CN 202310494008
(71) Applicant: GUANGZHOU MEDICAL UNIVERSITY, Guangzhou, Guangdong 511436 (CN)
(72) Inventor: ZHAO, Junfeng, Guangdong 511436 (CN); LIU, Tao, Guangdong 511436 (CN); PENG, Zejun, Guangdong 511436 (CN)
(74) Representative: IP Sextant s.r.l.
(86) International application number: PCT/CN2023/098911
(87) International publication number: WO 2024/229918

(57) **Abstract**

Provided is a method for synthesizing an amide and/or a polypeptide by using temporarily protected amino acids as amine components. The method uses an alkynyl amide compound as a condensation reagent, and low-cost unprotected amino acids as raw materials. The method comprises using a silanization reagent to temporarily protect an amino acid, and performing a condensation reaction. After the construction of a peptide bond is realized, a target polypeptide carboxylic acid can be obtained by means of simple acid treatment, and same can be directly used for condensation of a next amino acid.

## Description

### TECHNICAL FIELD

The present disclosure relates to the technical field of organic synthetic chemistry, and in particular relates to a method for synthesizing an amide and/or a polypeptide using a temporary protected amino acid as an amine component.

### BACKGROUND

Polypeptides and proteins are molecules with important functional activities formed by orderly connecting several natural α-amino acids through amide bonds (peptide bonds), and the construction of the amide bonds is the most basic chemical reaction in the synthesis of peptides and proteins. The method of constructing the amide bond is mainly to convert a carboxylic acid into a reactive intermediate such as acyl chloride, acid anhydride, activated ester and acyl azide through an activating reagent or a condensation agent, and then to react with another molecule of amine to form the amide bond. However, when the peptide bond is synthesized, since the amino acid contains both amino and carboxyl, the condensation of the two amino acids will produce various mixtures in any order, therefore the amino or carboxyl that do not require reaction must be temporarily protected with appropriate groups, and then a ligation reaction is performed to ensure directional synthesis. The commonly used amino acid has N-terminal protecting groups including fluorenylmethoxycarbonyl, tert-butoxycarbonyl, benzyloxycarbonyl and the like as well as C-terminal protecting groups including methyl ester, ethyl ester, tert-butyl ester, benzyl ester and the like. The introduction of these protecting groups requires additional reagents, and in addition, when these protecting groups are removed, a large excess of chemical reagents such as a strong acid, a strong base, and a palladium catalyst are required. The use of the amino acid protecting groups not only directly results in extremely low step economy and atom economy of peptide synthesis, but also increases the cost of peptide synthesis, causing serious pollution to the environment.

In 2016, some scholars designed and developed a new ynamide condensation agent for the synthesis of the amide bond and the peptide bond. This new condensing agent is easy to prepare, has good stability, small molecular weight, mild reaction conditions, and does not require any additives during use. More importantly, the α-chiral acid does not racemize in the condensing process, greatly improving the purity and yield of a product. However, this condensation agent still reacts with a carboxyl-protected amino acid as the amine component during the polypeptide synthesis. After the reaction is completed, the protected polypeptide is still obtained, and continuing to extend a peptide chain still requires a deprotection treatment, leading to a tedious polypeptide synthesis step, which is time-consuming and labor-consuming, and has a relatively high cost.

### SUMMARY

The present disclosure aims to solve at least one of the technical problems of complicated steps and low efficiency due to the use of amino acid protecting groups in the existing polypeptide synthesis technology. To this end, the object of the present disclosure is to provide a method for synthesizing an amide and/or a polypeptide using a temporary protected amino acid as an amine component. In the present disclosure, a silylation reagent is used to temporary protect the unprotected amino acids of the amino and carboxyl or the carboxyl in a peptide fragment, and then after the amide bond or the peptide bond is formed with the α-acyloxyenamide derivative of the carboxylic acid, the temporary protected carboxyl can be deprotected on-site in an acid treatment step to obtain a target carboxylic acid product, and then the building cycle for the next peptide bond is continued. On the other side, the ynamide condensation agent used in the present disclosure can effectively avoid racemization, that is, the condensing reagent or condensing method used when synthesizing the polypeptide using the unprotected amino acid in the related prior art can only synthesize a dipeptide, and a serious racemization will occur when synthesizing a tripeptide or a polypeptide including at least three amino acids. The method provided by the present disclosure avoids the racemization problem in the process of synthesizing the polypeptide, and this "temporary protection" strategy allows the three-step reaction of "protection-condensing-deprotection" to be carried out in one pot, the original three steps are combined into one step, to avoid using the large excess of chemical reagents, time, manpower and energy consumption required to pre-prepare the protected amino acids and remove these protecting groups, improve the step economy and atom economy of peptide synthesis, and greatly reduce the cost of peptide synthesis.

To achieve the foregoing object, the technical solution adopted by the present disclosure is as follows:
In a first aspect of the present disclosure, provided is a method for preparing an amide and/or a polypeptide, including the following steps: 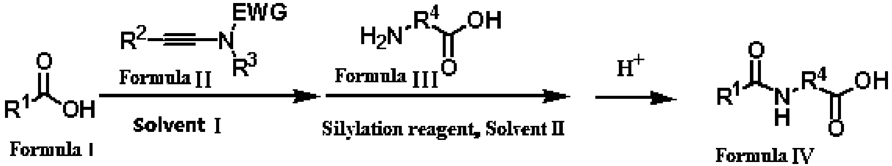
reacting a compound of formula I with a compound of formula II in a solvent I, and adding a compound of formula III, a silylation reagent and a solvent II to perform a nucleophilic substitution reaction, and then obtaining a compound of formula IV after acidification;
wherein R¹ is selected from alkyl, alkenyl, alkynyl, cycloalkyl, heterocycloalkyl, aryl, heteroaryl, an amino acid residual body lacking C-terminal carboxyl, and an amino acid derivative residual body or a polypeptide fragment lacking C-terminal carboxyl; R² is selected from hydrogen, deuterium, alkyl, cycloalkyl, heterocycloalkyl, aryl, heteroaryl, a silyl or an alkylsilyl; R³ is selected from alkyl, cycloalkyl, heterocycloalkyl, aryl and heteroaryl; R⁴ is selected from alkyl, cycloalkyl, heterocycloalkyl, aryl, heteroaryl, an amino acid residue or a polypeptide fragment; EWG (electron-withdrawing group) is selected from nitro, cyano, sulfonyl, arylsulfonyl, alkanoyl and phosphonyl; the alkyl, alkenyl, alkynyl, cycloalkyl, heterocycloalkyl, aryl, heteroaryl, silyl and alkylsilyl are optionally unsubstituted, or substituted by one or more R¹¹; and each R¹¹ is independently selected from halogen, hydroxyl, carbonyl and C₁-C₆ alkyl.

In some embodiments of the present disclosure, the aryl is selected from phenyl, o-tolyl, m-tolyl, p-tolyl, 2,4-xylyl, p-cumenyl, mesityl, 1-naphthyl, 2-naphthyl, 1-anthryl, 2-anthryl, 9-anthryl, 1-phenanthryl, 9-phenanthryl, 1-acenaphthenyl, 2-azulenyl, 1-pyrenyl, 2-triphenylene, o-biphenyl, m-biphenyl, p-biphenyl and terphenyl.

In some embodiments of the present disclosure, the heteroaryl is selected from a heterocyclyl having 1 or 2 nitrogen atoms, oxygen atoms or sulfur atoms, and is preferably 5 to 10 members, such as, but not limited to triazolyl, 3-oxadiazolyl, 2-furyl, 3-furyl, 2-thienyl, 3-thienyl, 1-pyrrolyl, 2-pyrrolyl, 3-pyrrolyl, 2-pyridyl, 3-pyridyl, 4-pyridyl, 2-pyrazinyl, 2-oxazolyl, 3-isoxazolyl, 2-thiazolyl, 3-isothiazolyl, 2-imidazolyl, 3-pyrazolyl, 2-quinolyl, 3-quinolyl, 4-quinolyl, 5-quinolyl, 6-quinolyl, 7-quinolyl, 8-quinolyl, 1-isoquinolyl, 2-quinoxalinyl, 2-benzofuranyl, 2-benzothienyl, N-indolyl and N-carbazolyl.

In some embodiments of the present disclosure, in R¹, the amino acid residual body lacking C-terminal carboxyl or the amino acid derivative residual body lacking C-terminal carboxyl refers to the remaining part of an amino acid or amino acid derivative after a C-terminal carboxyl group was removed. Preferably, the amino acid residual body lacking C-terminal carboxyl includes at least one of an α-amino acid residual body lacking C-terminal carboxyl, a β-amino acid residual body lacking C-terminal carboxyl, or a γ-amino acid residual body lacking C-terminal carboxyl. Preferably, the amino acid derivative residual body lacking C-terminal carboxyl includes the amino acid residual body lacking C-terminal carboxyl in which the amino group at C-terminal is protected, such as the amino acid residual body lacking C-terminal carboxyl in which the amino group at C-terminal is protected by N-alkoxycarbonyl and/or N-acyl. Preferably, α-amino acid is generally represented by 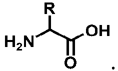 In R¹, the amino acid residual body lacking C-terminal carboxyl is 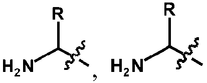 can be in R or S configuration. Specifically, the amino acid residual body lacking C-terminal carboxyl is selected from 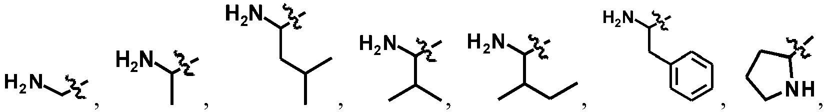 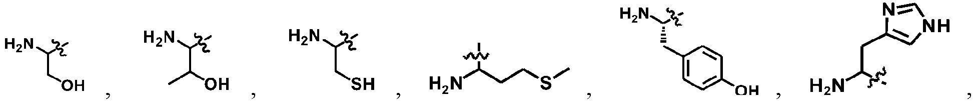 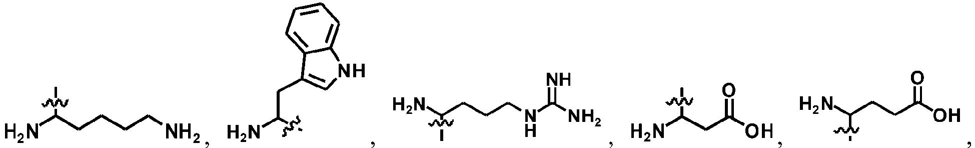 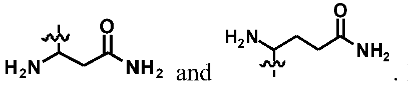 Preferably, the amino acid derivative residual body lacking C-terminal carboxyl is selected from an amino acid residual body lacking C-terminal carboxyl in which the amino group at C-terminal is protected by N-alkoxycarbonyl and/or N-acyl, among the above-mentioned amino acid residual body lacking C-terminal carboxyl.

In some embodiments of the present disclosure, when the solvent II is different from the solvent I, the method for preparing an amide and/or a polypeptide further includes removing the solvent I after the reaction and then performing the nucleophilic substitution reaction.

In some embodiments of the present disclosure, an acidifying agent for acidification includes an organic acid and/or an inorganic acid.

The term "acidifying agent" may mean any material that lowers the pH of a solution by being dissolved in water. In some embodiments, the acidifying agent may be an inorganic acid and/or an organic acid that can be dissolved in water to lower the pH of the solution to 5 or lower (such as, pH ≤4, pH≤3, pH≤2, pH≤1).

The inorganic acid may include hydrochloric acid, hydrobromic acid, hydroiodic acid, phosphoric acid, potassium dihydrogen phosphate, sodium dihydrogen phosphate, sulfonic acid, sulfamic acid, sulfuric acid, nitric acid, or any combination thereof, and however the embodiment is not limited thereto. The organic acid may include citric acid, lactic acid, tartaric acid, fumaric acid, trifluoroacetic acid, phthalic acid, acetic acid, oxalic acid, malonic acid, adipic acid, phytic acid, succinic acid, glutaric acid, maleic acid, malic acid, mandelic acid, ascorbic acid, benzoic acid, methanesulfonic acid, p-toluenesulfonic acid, salicylic acid, capric acid, caproic acid, caprylic acid, lauric acid, arachidic acid, erucic acid, linoleic acid, linolenic acid, oleic acid, palmitic acid, myristic acid, edisilic acid, stearic acid or any combination thereof. However, the embodiment is not limited thereto.

According to a second aspect of the present disclosure, provided is another method for preparing an amide and/or a polypeptide, including the following steps: 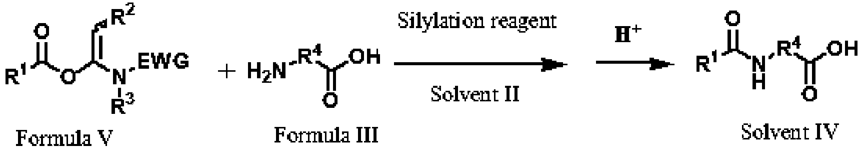
dissolving a compound of formula V, a compound of formula III, and a silylation reagent in a solvent II to perform a nucleophilic substitution reaction, and then preparing a compound of formula IV after acidification;
wherein R¹, R², R³ and R⁴ are defined as mentioned above.

In the present disclosure, since the compound of formula III (a compound with both amino and carboxyl) exists in the form of a compound A of formula III (a zwitter-ion in the molecule) under a neutral condition, under a normal condition, the amino in the compound A of formula III is not nucleophilic is difficult to perform a condensation reaction, the compound A of formula III can be temporary protected by using the silylation reagent and the carboxyl thereof temporary forms the compound B of formula III-B (a silicon ester compound), and at the same time the amino thereof is free, then the nucleophilic substitution reaction can occur with the found of formula V to form an amide bond/peptide bond, thereby preparing an amide or polypeptide compound of formula IV, and at the same time forming a by-product compound of formula VI. The reaction mechanism is as follows:

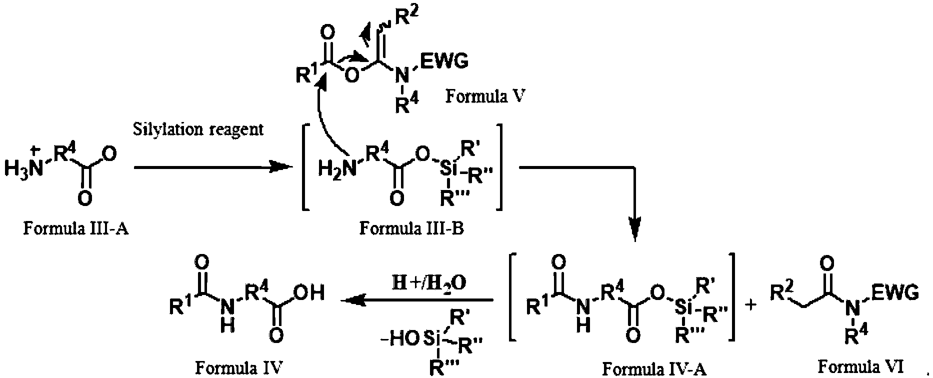

In some embodiments of the present disclosure, the silylation reagent includes at least one of N-trimethylsilylacetamide, N-methyl-N-trimethylsilylacetamide, N-trimethylsilylpyrrolidone, N-(tertbutyldimethylsilyl)-N-methyltrifluoroacetamide, N,O-bis(tert-butyldimethylsilyl)acetamide, N,O-bistrimethylsilylacetamide, N,O-bis(trimethylsilyl)trifluoroacetamide, N-methyl-N-(trimethylsilyl)trifluoroacetamide, hexamethyldisilylurea, trimethylsilimidazole, dimethyl dichlorosilane, trimethylchlorosilane and tert-butyldimethylchlorosilane.

In some embodiments of the present disclosure, a molar ratio of the silylation reagent to the compound of formula III is (0.5-20): 1, preferably (1-15): 1, and further preferably (2-10): 1.

In some embodiments of the present disclosure, the compound of formula III is an organic compound having both amino and carboxyl. Preferably, the compound of formula III includes any one of an amino acid compound, a polypeptide compound with free amino and carboxyl or other compounds with both amino and carboxyl. Further preferably, the compound of formula III includes any one of α-amino acid, β-amino acid, γ-amino acid or a polypeptide compound in which neither the amino nor the carboxyl is protected. Still further preferably, the compound of formula II includes any one of glycine, alanine, leucine, isoleucine, proline, valine, phenylalanine, tyrosine, serine, threonine, cysteine, methionine, aspartic acid, glutamic acid, asparagine, glutamine, tryptophan, histidine, lysine, arginine, citrulline or derivative compounds of the above amino acids. It should be understood that the twenty-one amino acids included in the compound of formula III can all be natural α-amino acids in the L-configuration and/or their corresponding α-amino acids in the D-configuration.

In some embodiments of the present disclosure, the solvent II includes at least one of acetonitrile, diethyl ether, ethylene glycol dimethyl ether, ethylene glycol diethyl ether, dioxane, dimethyl sulfoxide, N,N-dimethylformamide, N,N-dimethylacetamide, N-methylpyrrolidone, toluene, m-xylene, dichloromethane, 1,2-dichloroethane or chloroform.

In some embodiments of the present disclosure, the temperature of the nucleophilic substitution reaction is -20°C to 150°C, preferably 0°C to 90°C, and further preferably 5°C to 50°C.

In some embodiments of the present disclosure, a method for preparing an amide and/or a polypeptide includes the following steps: dissolving the compound of formula III and the silylation reagent in solvent II, then adding the compound of formula V to perform the nucleophilic substitution reaction, and preparing the compound of formula IV after acidification.

In some embodiments of the present disclosure, a method for preparing an amide and/or a polypeptide includes the following steps: stirring the compound of formula III and the silylation reagent stirred for 5 min to 24 hours and dissolving the mixture in the solvent II, then adding the compound of formula V to perform the nucleophilic substitution reaction, and preparing the compound of formula IV after acidification.

In some embodiments of the present disclosure, a method for preparing an amide and/or a polypeptide includes a step of purifying the product after the nucleophilic substitution reaction, and the purification includes at least one of extraction, recrystallization or column chromatography.

In some embodiments of the present disclosure, a method for preparing a compound of formula V includes the following steps: 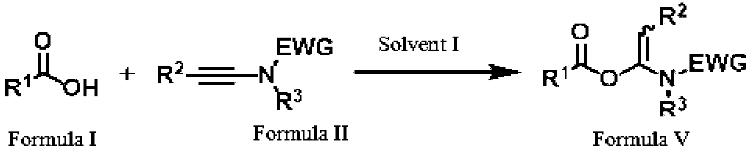
dissolving a compound of formula I and a compound of formula II in a solvent I to obtain a mixture, and reacting the mixture under stirring to prepare a compound of formula V;
wherein R¹, R², R³ and EWG are defined as mentioned above.

In some embodiments of the present disclosure, the solvent I includes at least one of dichloromethane, chloroform, 1,2-dichloroethane, diethyl ether, toluene, acetonitrile, methanol or ethanol.

In some embodiments of the present disclosure, the compound of formula I is a carboxylic acid compound. Preferably, the compound of formula I includes at least one of fatty acid, aromatic acid, heterocyclic acid, acetylenic acid, olefine acid, N-acylamino acid, N-alkoxycarbonyl amino acid or polypeptide carboxylic acid. Further preferably, the compound of formula I includes at least one of N-benzyloxycarbonyl amino acid, N-tert-butoxycarbonyl amino acid, N-fluorenylmethoxycarbonyl amino acid, N-acetyl amino acid or polypeptide carboxylic acid.

In some embodiments of the present disclosure, a molar ratio of the compound of formula I to the compound of formula II is 1: (1-5), preferably 1: (1.1-4), and further preferably 1: (1.2-3).

In some embodiments of the present disclosure, the temperature of the stirring reaction is -20°C to 90°C, preferably 0°C to 50°C.

In some embodiments of the present disclosure, a method for preparing an amide and/or a polypeptide includes the following steps: 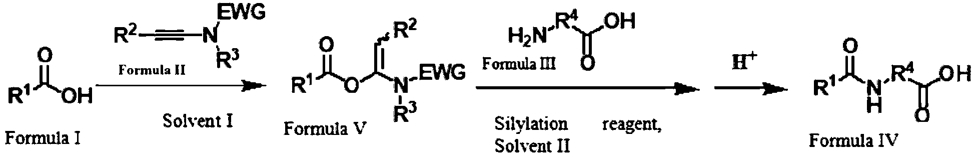
S1: dissolving a compound of formula I and a compound of formula II in a solvent I to obtain a mixture, and reacting the mixture under stirring to prepare a compound of formula V; and
S2: dissolving the compound of formula V, the compound of formula III and the silylation reagent in the solvent II to perform a nucleophilic substitution reaction, and then preparing a compound of formula IV after acidification;
wherein R¹, R², R³, R⁴ and EWG are defined as mentioned above.

According to a third aspect of the present disclosure, provided is a use of the method for preparing the amide and/or polypeptide in preparing the polypeptide.

The beneficial effects of the present disclosure are as follows:
In the method for preparing an amide and/or a polypeptide provided by the present disclosure, the ynamide compound is used as the condensation agent, a low-cost unprotected amino acid is used as the raw material, and the silylation reagent is used to temporary protect the amino acid to perform the condensation reaction, so that "protection-condensation-deprotection" are carried out in one pot, and the three steps of reaction are combined into one step. After the construction of the peptide bond is achieved, the target polypeptide carboxylic acid can be obtained by a simple acid treatment, which can be directly used for the condensation of the next amino acid. At the same time, based on the characteristics of ynamide condensation agent, this method can also completely avoid the formation of racemic by-products, to ensure the yield and purity of the product. This method saves a large number of reagents and solvents used in the traditional methods of "protecting groups on amino acids" and "product deprotection", reduces the generation of a large amount of chemical waste, saves time, human resources and energy consumption, greatly reduces the cost of peptide synthesis, improves the atom economy and step economy of peptide synthesis, effectively achieves energy saving and emission reduction, greatly improves the efficiency of peptide synthesis, and has a broad industrial application prospect.

### DETAILED DESCRIPTION

The content of the present disclosure will be further described in detail below through specific examples. Unless otherwise specified, the raw materials, reagents or devices used in the examples and comparative examples can be obtained from conventional commercial sources, or can be obtained through existing technical methods. Unless otherwise stated, assays or testing methods are routine in the art.

### Example 1

A polypeptide of Formula 1 was prepared in the example. A specific process was as follows:

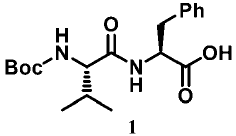

Boc-Val-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by Thin Layer Chromatography (TLC). When the raw acid was completely consumed, the dichloromethane was removed by distillation under reduced pressure and transferred to a system that was mixed with H-Phe-OH (1 mmol), N-trimethylsilylacetamide (1 mmol) and 3 mL of N,N-dimethylformamide in advance for reaction, and the reaction process was monitored by TLC. After the reaction was completed, 15 mL of water was added to acidify to pH=2 with 10% citric acid. An aqueous phase was extracted twice with ethyl acetate, an organic phase was combined, washed once with water, washed once with a saturated salt solution, and dried with anhydrous sodium sulfate. The solvent was removed by distillation under reduced pressure, recrystallized with diethyl ether, and the solid was filtered and collected to obtain a pure product, with a white solid, *dr >* 99:1, and a yield of 94%.

Magnetic resonance imaging experimental data and mass spectrometry experimental data of products were as follows:
**¹H NMR** (400 MHz, DMSO) δ 8.04 (d, J = 7.8 Hz, 1H), 7.30 - 7.16 (m, 5H), 6.54 (d, J = 9.2 Hz, 1H), 4.47 (dd, J = 13.3, 8.3 Hz, 1H), 3.78 (t, J = 8.0 Hz, 1H), 3.06 (dd, J = 13.9, 5.0 Hz, 1H), 2.90 (dd, J = 13.9, 9.1 Hz, 1H), 1.97 - 1.77 (m, 1H), 1.38 (s, 9H), 0.83 - 0.71 (m, 6H).
**¹³C NMR** (100 MHz, DMSO) δ = 172.8, 171.3, 155.2, 137.4, 129.1, 128.1, 126.4, 78.0, 59.7, 53.2, 36.8, 30.5, 28.2, 19.1, 18.1.
**HRMS (ESI)** m/z calcd. for C₁₉H₂₉N₂O₅ [M+H]⁺: 365.2071, found: 365.2077.

### Example 2

A polypeptide of Formula 2 was prepared in the example. A specific process was as follows:

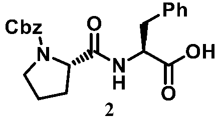

Cbz-Pro-OH (0.5 mmol), N-methyl-N-methylsulfonyl ethynylamine (0.55 mmol) were taken to be dissolved in 5 mL of dichloromethane, a stirring reaction was performed at a room temperature, and the reaction process was monitored by TLC. When the raw acid was completely consumed, the mixture was transferred to a system that was mixed with H-Phe-OH (1 mmol), N,O-bistrimethylsilylacetamide (1 mmol) and 3 mL of dichloroethane in advance for reaction, and the reaction process was monitored by TLC. After the reaction was completed, 15 mL of water was added to acidify to pH=2 with 2 M of hydrochloric acid. An aqueous phase was extracted twice with ethyl acetate, an organic phase was combined, washed once with water, washed once with a saturated salt solution, and dried with anhydrous sodium sulfate. The solvent was removed by distillation under reduced pressure and subjected to column chromatography isolation to obtain a pure product, with a white solid, *dr >* 99:1, and a yield of 93%.

Magnetic resonance imaging experimental data and mass spectrometry experimental data of products were as follows:
**¹H NMR** (400 MHz, DMSO-*d₆*) δ 8.38 - 8.03 (m, 1H), 7.42 - 7.11 (m, 10H), 5.12 - 5.02 (m, 1H), 5.01 - 4.82 (m, 1H), 4.53 - 4.40 (m, 1H), 4.23 (d, J = 8.1 Hz, 1H), 3.40 - 3.29 (m, 2H), 3.12 - 3.00 (m, 1H), 2.99 - 2.84 (m, 2H), 2.13 - 1.96 (m, 1H), 1.80 - 1.59 (m, 3H).
**¹³C NMR** (100 MHz, DMSO) δ = 172.9, 172.8, 172.1, 171.8, 154.2, 153.8, 137.6, 137.5, 137.1, 129.2, 129.0, 128.4, 128.2, 128.1, 128.1, 127.8, 127.5, 127.0, 126.4, 126.3, 65.9, 65.7, 59.7, 59.3, 53.5, 53.1, 47.1, 46.5, 36.6, 31.0, 29.7, 23.7, 22.8.
**HRMS (ESI)** m/z calcd. for C₂₂H₂₅N₂O₅ [M+H]⁺: 397.1758, found: 397.1764.

### Example 3

A polypeptide of Formula 3 was prepared in the example. A specific process was as follows:

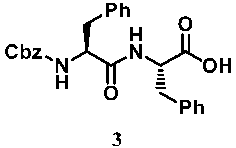

Cbz-Phe-OH (0.5 mmol), N-methyl-N-p-toluenesulfonyl ethynylamine (0.5 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that was mixed with H-Phe-OH (1 mmol), N,O-bistrimethylsilylacetamide (1 mmol) and 3 mL of N,N-dimethylformamide in advance for reaction, and the reaction process was monitored by TLC. After the reaction was completed, 15 mL of water was added to acidify to pH=2 with 2 M of hydrochloric acid. An aqueous phase was extracted twice with ethyl acetate, an organic phase was combined, washed once with water, washed once with a saturated salt solution, and dried with anhydrous sodium sulfate. The solvent was removed by distillation under reduced pressure and subjected to column chromatography isolation to obtain a pure product, with a white solid, *dr >* 99:1, and a yield of 95%.

Magnetic resonance imaging experimental data and mass spectrometry experimental data of products were as follows:
**¹H NMR** (400 MHz, DMSO-*d₆*) δ 8.28 (d, *J* = 7.7 Hz, 1H), 7.46 (d, *J* = 8.7 Hz, 1H), 7.37 - 7.17 (m, 15H), 4.95 (s, 2H), 4.52 (dd, *J =* 13.1, 7.7 Hz, 1H), 4.37 - 4.28 (m, 1H), 3.12 (dd, *J =* 13.8, 5.0 Hz, 1H), 3.03 - 2.90 (m, 2H), 2.78 - 2.67 (m, 1H).
**¹³C NMR** (100 MHz, DMSO-*d₆*) δ 173.2, 172.0, 156.2, 138.5, 137.8, 137.5, 129.7, 128.7, 128.7, 128.5, 128.1, 127.9, 126.9, 126.7, 65.7, 56.5, 53.9, 37.9, 37.2.
**HRMS (ESI)** m/z calcd. for C₂₆H₂₇N₂O₆ [M+H]⁺: 447.1914, found: 447.1919.

### Example 4

A polypeptide of Formula 4 was prepared in the example. A specific process was as follows:

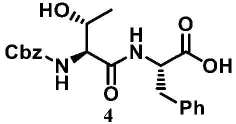

Cbz-Thr-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that was mixed with H-Phe-OH (1 mmol), hexamethyldisilylurea (1 mmol) and 3 mL of acetonitrile in advance for reaction, and the reaction process was monitored by TLC. After the reaction was completed, 15 mL of water was added to acidify to pH=2 with 2 M of hydrochloric acid. An aqueous phase was extracted twice with ethyl acetate, an organic phase was combined, washed once with water, washed once with a saturated salt solution, and dried with anhydrous sodium sulfate. The solvent was removed by distillation under reduced pressure, recrystallized with diethyl ether, and the solid was filtered and collected to obtain a pure product, with a white solid, *dr >* 99:1, and a yield of 95%.

Magnetic resonance imaging experimental data and mass spectrometry experimental data of products were as follows:
**¹H NMR** (400 MHz, DMSO-*d₆*) δ 8.06 (d, *J =* 7.7 Hz, 1H), 7.41 - 7.17 (m, 10H), 6.95 (d, *J*= 8.7 Hz, 1H), 5.05 (s, 2H), 4.48 (q, *J =* 7.3 Hz, 1H), 3.96 (dd, *J =* 8.2, 5.2 Hz, 1H), 3.88 - 3.76 (m, 1H), 3.05 (dd, *J =* 13.7, 5.0 Hz, 1H), 2.93 (dd, *J =* 13.6, 8.1 Hz, 1H), 1.01 (d, *J =* 6.1 Hz, 3H).
**¹³C NMR** (100 MHz, DMSO-*d₆*) δ 172.6, 170.0, 156.0, 137.3, 137.0, 129.2, 128.3, 128.1, 127.8, 127.7, 126.4, 66.8, 65.5, 60.5, 53.3, 36.9, 19.6.
**HRMS (ESI)** m/z calcd. for C₂₁H₂₄N₂NaO₆ [M+Na]⁺: 423.1527, found: 423.1524.

### Example 5

A polypeptide of Formula 5 was prepared in the example. A specific process was as follows:

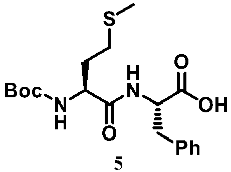

Boc-Met-OH (0.5 mmol), N-methyl-N-p-toluenesulfonyl ethynylamine (0.5 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that was mixed with H-Phe-OH (1 mmol), N-Trimethylsilylimidazole (1 mmol) and 3 mL of acetonitrile in advance for reaction, and the reaction process was monitored by TLC. After the reaction was completed, 15 mL of water was added to acidify to pH=3 with 10% of citric acid. An aqueous phase was extracted twice with ethyl acetate, an organic phase was combined, washed once with water, washed once with a saturated salt solution, and dried with anhydrous sodium sulfate. The solvent was removed by distillation under reduced pressure and subjected to column chromatography isolation to obtain a pure product, with a white solid, dr>99:1, and a yield of 97%.

Magnetic resonance imaging experimental data and mass spectrometry experimental data of products were as follows:
**¹H NMR** (400 MHz, DMSO-*d₆*) δ 7.97 (d, *J =* 7.8 Hz, 1H), 7.34 - 7.16 (m, 5H), 6.91 (d, *J* = 8.2 Hz, 1H), 4.46 (q, *J =* 8.0 Hz, 1H), 4.01 (q, *J =* 7.9 Hz, 1H), 3.07 (dd, *J =* 13.8, 4.9 Hz, 1H), 2.92 (dd, *J =* 13.9, 8.7 Hz, 1H), 2.45 - 2.29 (m, 2H), 2.01 (s, 3H), 1.85 - 1.64 (m, 2H), 1.37 (s, 9H).
**¹³C NMR** (100 MHz, DMSO-*d₆*) δ 172.8, 171.6, 155.2, 137.3, 129.2, 128.1, 126.4, 78.2, 53.6, 53.2, 36.7, 31.9, 29.6, 28.2, 14.6.
**HRMS (ESI)** m/z calcd. for C₁₉H₂₈N₂NaO₅S [M+Na]⁺: 419.1611, found: 419.1609.

### Example 6

A polypeptide of Formula 6 was prepared in the example. A specific process was as follows:

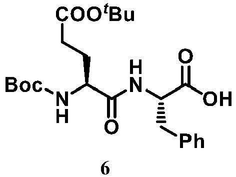

Boc-Glu(O*^{t}*Bu)-OH (0.5 mmol), N-methyl-N-methylsulfonyl ethynylamine (0.5 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that was mixed with H-Phe-OH (1 mmol), N, O-bis(trimethylsilyl)trifluoroacetamide (1 mmol) and 3 mL of dimethyl sulfoxide in advance for reaction, and the reaction process was monitored by TLC. After the reaction was completed, 15 mL of water was added to acidify to pH=3 with 10% of citric acid. An aqueous phase was extracted twice with ethyl acetate, an organic phase was combined, washed once with water, washed once with a saturated salt solution, and dried with anhydrous sodium sulfate. The solvent was removed by distillation under reduced pressure and subjected to column chromatography isolation to obtain a pure product, with a white solid, *dr >* 99:1, and a yield of 94%.

Magnetic resonance imaging experimental data and mass spectrometry experimental data of products were as follows:
**¹H NMR** (400 MHz, DMSO- *d₆*) δ 7.95 (d, *J =* 7.9 Hz, 1H), 7.36 - 7.10 (m, 5H), 6.82 (d, *J =* 8.3 Hz, 1H), 4.52 - 4.40 (m, 1H), 4.01 - 3.85 (m, 1H), 3.07 (dd, *J =* 13.8, 4.8 Hz, 1H), 2.92 (dd, *J =* 13.9, 8.8 Hz, 1H), 2.16 (t, *J =* 7.6 Hz, 2H), 1.85 - 1.72 (m, 1H), 1.70 - 1.58 (m, 1H), 1.40 (s, 9H), 1.37 (s, 9H).
**¹³C NMR** (100 MHz, DMSO-*d₆*) δ 172.7, 171.7, 171.5, 155.1, 137.3, 129.2, 128.1, 126.4, 79.5, 78.2, 53.5, 53.2, 36.7, 31.2, 28.1, 27.8, 27.4.
**HRMS (ESI)** m/z calcd. for C₂₃H₃₄N₂O₇ [M+H]⁺: 451.2439, found: 451.2445.

### Example 7

A polypeptide of Formula 7 was prepared in the example. A specific process was as follows:

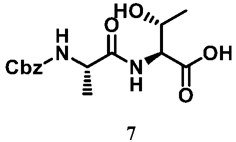

Cbz-Ala-OH (0.5 mmol), N-methyl-N-p-toluenesulfonyl ethynylamine (0.55 mmol) were taken to be dissolved in 5 mL of trichloromethane, a stirring reaction was performed at a room temperature, and the reaction process was monitored by TLC. When the raw acid was completely consumed, the mixture was transferred to a system that was mixed with H-Thr-OH (1 mmol), N,O-bistrimethylsilylacetamide (2 mmol) and 3 mL of trichloromethane in advance for reaction, and the reaction process was monitored by TLC. After the reaction was completed, 15 mL of water was added to acidify to pH=2 with 2 M of hydrochloric acid. An aqueous phase was extracted twice with ethyl acetate, an organic phase was combined, washed once with water, washed once with a saturated salt solution, and dried with anhydrous sodium sulfate. The solvent was removed by distillation under reduced pressure, recrystallized with ethyl acetate and diethyl ether, and the solid was filtered and collected to obtain a pure product, with a white solid, *dr >* 99:1, and a yield of 92%.

Magnetic resonance imaging experimental data and mass spectrometry experimental data of products were as follows:
**¹H NMR** (400 MHz, DMSO-*d₆*) δ 7.67 (d, J = 8.6 Hz, 1H), 7.53 (d, J = 7.7 Hz, 1H), 7.39 - 7.26 (m, 5H), 5.03 (s, 2H), 4.25 - 4.14 (m, 3H), 1.24 (d, J = 7.1 Hz, 3H), 1.06 (d, J = 6.1 Hz, 3H).
**¹³C NMR** (100 MHz, DMSO-*d₆*) δ 173.0,172.2, 155.8, 137.1, 128.5, 127.9, 127.8, 66.5, 65.5, 57.5, 50.2, 20.4, 18.2.
**HRMS (ESI)** m/z calcd. for C₁₃H₂₀N₂O₆ [M+Na]⁺: 347.1214, found: 347.1221.

### Example 8

A polypeptide of Formula 8 was prepared in the example. A specific process was as follows:

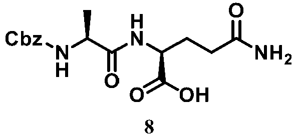

Cbz-Ala-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that was mixed with H-Gln-OH (1 mmol), N,O-bistrimethylsilylacetamide (2 mmol) and 3 mL of N,N-dimethylacetamide in advance for reaction, and the reaction process was monitored by TLC. After the reaction was completed, 15 mL of water was added to acidify to pH=1 with 2 M of hydrochloric acid. An aqueous phase was extracted twice with ethyl acetate, an organic phase was combined, washed once with water, washed once with a saturated salt solution, and dried with anhydrous sodium sulfate. The solvent was removed by distillation under reduced pressure, recrystallized with dichloromethane and diethyl ether, and the solid was filtered and collected to obtain a pure product, with a white solid, *dr* >99:1, and a yield of 95%.

Magnetic resonance imaging experimental data and mass spectrometry experimental data of products were as follows:
**¹H NMR** (400 MHz, DMSO-*d₆*) δ 8.13 (d, *J =* 7.6 Hz, 1H), 7.59 - 7.12 (m, 7H), 6.79 (s, 1H), 5.09 - 4.95 (m, 2H), 4.22 - 4.05 (m, 2H), 2.22 - 2.07 (m, 2H), 2.01 - 1.91 (m, 1H), 1.86 - 1.73 (m, 1H), 1.22 (d, *J =* 7.1 Hz, 3H).
**¹³C NMR** (100 MHz, DMSO-*d₆*) δ 173.6, 173.3, 172.7, 155.7, 137.1, 128.4, 127.8, 127.8, 65.4, 51.6, 49.9, 31.4, 27.0, 18.2.
**HRMS (ESI)** m/z calcd. for C₁₆H₂₁N₃NaO₆ [M+Na]⁺: 374.1323, found: 374.1321.

### Example 9

A polypeptide of Formula 9 was prepared in the example. A specific process was as follows:

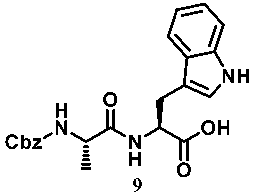

Cbz-Ala-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that was mixed with H-Trp-OH (1 mmol), hexamethyldisilylurea (1 mmol) and 3 mL of N-methylpyrrolidone in advance for reaction, and the reaction process was monitored by TLC. After the reaction was completed, 15 mL of water was added to acidify to pH=1 with 2 M of hydrochloric acid. An aqueous phase was extracted twice with ethyl acetate, an organic phase was combined, washed once with water, washed once with a saturated salt solution, and dried with anhydrous sodium sulfate. The solvent was removed by distillation under reduced pressure and subjected to column chromatography isolation to obtain a pure product, with a white solid, *dr >* 99:1, and a yield of 95%.

Magnetic resonance imaging experimental data and mass spectrometry experimental data of products were as follows:
**¹H NMR** (400 MHz, DMSO-*d₆*) δ 10.89 (s, 1H), 8.12 - 8.03 (m, 1H), 7.57 (t, *J* = 6.0 Hz, 1H), 7.44 (dd, *J =* 8.1, 4.1 Hz, 1H), 7.34 (d, *J* = 15.7 Hz, 8H), 7.20 (s, 1H), 7.07 (s, 1H), 6.98 (s, 1H), 5.13 - 4.94 (m, 3H), 4.54 (s, 1H), 4.15 (q, *J =* 5.8, 4.9 Hz, 2H), 3.22 (d, *J* = 14.3 Hz, 2H), 3.17 - 3.05 (m, 2H), 1.26 - 1.19 (m, 7H).
**¹³C NMR** (100 MHz, DMSO-*d₆*) δ 173.3, 172.6, 155.7, 137.1, 136.1, 128.4, 127.8, 127.8, 127.4, 123.8, 121.0,118.4, 118.3, 111.4, 109.7, 65.5, 53.0, 50.0, 27.1, 18.3.
**HRMS (ESI)** m/z calcd. for C₂₂H₂₃N₃NaO₅ [M+Na]⁺: 432.1530, found: 432.1530.

### Example 10

A polypeptide of Formula 10 was prepared in the example. A specific process was as follows:

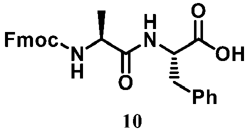

Fmoc-Ala-OH (0.5 mmol), N-methyl-N-p-toluenesulfonyl ethynylamine (0.55 mmol) were taken to be dissolved in 3 mL of dichloromethane, a stirring reaction was performed at a room temperature, and the reaction process was monitored by TLC. When the raw acid was completely consumed, the mixture was transferred to a system that was mixed with H-Phe-OH (1 mmol), N-methyl-N-trimethylsilylacetamide (1 mmol) and 3 mL of dichloromethane in advance for reaction, and the reaction process was monitored by TLC. After the reaction was completed, 15 mL of water was added to acidify to pH=1 with 2 M of hydrochloric acid. An aqueous phase was extracted twice with ethyl acetate, an organic phase was combined, washed once with water, washed once with a saturated salt solution, and dried with anhydrous sodium sulfate. The solvent was removed by distillation under reduced pressure and subjected to column chromatography isolation to obtain a pure product, with a white solid, *dr >* 99:1, and a yield of 93%.

Magnetic resonance imaging experimental data and mass spectrometry experimental data of products were as follows:
**¹H NMR** (400 MHz, DMSO-*d₆*) δ 8.02 (d, *J =* 7.5 Hz, 1H), 7.89 (d, *J =* 7.5 Hz, 2H), 7.74 (d, *J* = 6.8 Hz, 1H), 7.48 (d, *J =* 7.7 Hz, 1H), 7.42 (t, *J =* 7.4 Hz, 2H), 7.33 (t, *J =* 7.3 Hz, 2H), 7.27 - 7.16 (m, 4H), 4.50 - 4.39 (m, 1H), 4.26 (d, *J* = 6.1 Hz, 1H), 4.24 - 4.18 (m, 1H), 4.12 - 4.06 (m, 1H), 3.07 (dd, *J =* 13.7, 4.8 Hz, 1H), 2.93 (dd, *J =* 13.7, 8.4 Hz, 1H), 1.20 (d, *J =* 7.1 Hz, 3H).
**¹³C NMR** (100 MHz, DMSO-*d₆*) δ 172.8, 172.4, 155.6, 143.9, 143.8, 140.7, 137.4, 129.2, 128.1, 127.6, 127.1, 126.3, 125.3, 120.1, 65.7, 53.5, 49.9, 46.6, 36.7, 18.2.
**HRMS (ESI)** m/z calcd. for C₂₂H₂₃N₃NaO₅ [M+Na]⁺: 432.1530, found: 432.1530.

### Example 11

A polypeptide of Formula 11 is prepared in the example. A specific process is as follows:

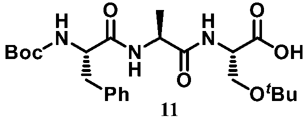

Boc-Phe-Ala-OH (0.5 mmol), N-methyl-N-p-toluenesulfonyl ethynylamine (0.5 mmol) were taken to be dissolved in 5 mL of dichloromethane, a stirring reaction was performed at a room temperature, and the reaction process was monitored by TLC. When the raw acid was completely consumed, the mixture was transferred to a system that was mixed with H- Ser(O*^{t}*Bu)-OH (1 mmol), N-trimethylsilylpyrrolidone (1 mmol) and 5 mL of 1,2-dichloroethane in advance for reaction, and the reaction process was monitored by TLC. After the reaction was completed, 15 mL of water was added to acidify to pH=3 with 10% of citric acid. An aqueous phase was extracted twice with dichloromethane, an organic phase was combined, washed once with water, washed once with a saturated salt solution, and dried with anhydrous sodium sulfate. The solvent was removed by distillation under reduced pressure, recrystallized with diethyl ether, and the solid was filtered and collected to obtain a pure product, with a white solid, *dr >* 99:1, and a yield of 94%.

Magnetic resonance imaging experimental data and mass spectrometry experimental data of products were as follows:
**¹H NMR** (400 MHz, DMSO-*d₆*) δ 8.04 *(d, J=* 7.4 Hz, 1H), 7.94 (d, *J* = 7.9 Hz, 1H), 7.32 - 7.15 (m, 5H), 6.94 (d, *J =* 8.5 Hz, 1H), 4.49 - 4.41 (m, 1H), 4.40 - 4.30 (m, 1H), 4.22 - 4.11 (m, 1H), 3.69 - 3.60 (m, 1H), 3.54 - 3.48 (m, 1H), 3.05 - 2.94 (m, 1H), 2.78 - 2.65 (m, 1H), 1.29 (s, 9H), 1.24 (d, *J =* 6.8 Hz, 3H), 1.11 (s, 9H)
**¹³C NMR** (100 MHz, DMSO-*d₆*) δ 172.1, 171.4, 171.3, 155.2, 138.2, 129.2, 127.9, 126.1, 78.0, 72.8, 61.5, 55.6, 52.8, 47.8, 37.4, 28.1, 27.1, 18.4.
**HRMS (ESI)** m/z calcd. for C₂₄H₃₈N₃O₇ [M+H]⁺: 480.2704, found: 480.2710.

### Example 12

A polypeptide of Formula 12 was prepared in the example. A specific process was as follows:

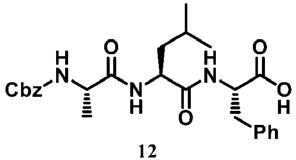

Cbz-Ala-Leu-OH (0.5 mmol), N-methyl-N-p-toluenesulfonyl ethynylamine (0.5 mmol) were taken to be dissolved in 5 mL of dichloromethane, a stirring reaction was performed at a room temperature, and the reaction process was monitored by TLC. When the raw acid was completely consumed, the mixture was transferred to a system that was mixed with H-Phe-OH (1 mmol), N,O-bistrimethylsilylacetamide (1 mmol) and 5 mL of dichloromethane in advance for reaction, and the reaction process was monitored by TLC. After the reaction was completed, 15 mL of water was added to acidify to pH=1 with 2 M of hydrochloric acid. An aqueous phase was extracted twice with dichloromethane, an organic phase was combined, washed once with water, washed once with a saturated salt solution, and dried with anhydrous sodium sulfate. The solvent was removed by distillation under reduced pressure, recrystallized with dichloromethane and diethyl ether, and the solid was filtered and collected to obtain a pure product, with a white solid, *dr >* 99:1, and a yield of 91%.

Magnetic resonance imaging experimental data and mass spectrometry experimental data of products were as follows:
**¹H NMR** (400 MHz, DMSO-*d₆*) δ 8.04 (d, *J =* 7.6 Hz, 1H), 7.86 (d, *J =* 8.3 Hz, 1H), 7.43 (d, *J* = 7.6 Hz, 1H), 7.37 - 7.17 (m, 10H), 5.02 (s, 2H), 4.47 - 4.41 (m, 1H), 4.36 - 4.30 (m, 1H), 4.10 - 4.04 (m, 1H), 3.07 (dd, *J =* 13.9, 5.2 Hz, 1H), 2.93 (dd, *J =* 13.8, 8.5 Hz, 1H), 1.67 - 1.50 (m, 1H), 1.43 (d, *J =* 6.7 Hz, 1H), 1.17 (d, *J =* 7.0 Hz, 3H), 0.90 - 0.76 (m, 6H).
**¹³C NMR** (100 MHz, DMSO-*d₆*) δ 1172.8, 172.2, 171.9, 155.7, 137.5, 137.1, 129.2, 128.4, 128.2, 127.8, 127.8, 126.4, 65.4, 53.4, 51.0, 50.1, 41.1, 36.7, 24.1, 23.1, 21.8, 18.2.
**HRMS (ESI)** m/z calcd. For C₂₆H₃₄N₃O₆ [M+H]⁺: 484.2442, found: 284.2448.

### Example 13

A polypeptide of Formula 13 was prepared in the example. A specific process was as follows:

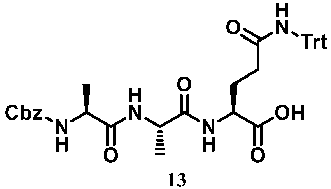

Cbz-Ala-Ala-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the mixture was transferred to a system that was mixed with H-Gln(Trt)-OH (1 mmol), N,O-bistrimethylsilylacetamide (2 mmol) and 5 mL of dichloromethane in advance for reaction, and the reaction process was monitored by TLC. After the reaction was completed, 15 mL of water was added to acidify to pH=3 with 10% of citric acid. An aqueous phase was extracted once with ethyl acetate and washed twice with water, an organic phase was washed once with a saturated salt solution and dried with anhydrous sodium sulfate. The solvent was removed by distillation under reduced pressure, recrystallized with dichloromethane and diethyl ether, and the solid was filtered and collected to obtain a pure product, with a light yellow solid, *dr >* 99:1, and a yield of 95%.

Magnetic resonance imaging experimental data and mass spectrometry experimental data of products were as follows:
**¹H NMR** (400 MHz, DMSO-*d₆*) δ 8.59 (s, 1H), 8.05 (d, *J =* 8.2 Hz, 1H), 7.93 (t, *J* = 6.5 Hz, 1H), 7.44 (t, *J =* 5.9 Hz, 1H), 7.38 - 7.14 (m, 22H), 5.06 - 4.97 (m, 2H), 4.37 - 4.29 (m, 1H), 4.22 - 4.15 (m, 1H), 4.11 - 4.05 (m, 1H), 2.41 - 2.29 (m, 2H), 1.99 - 1.88 (m, 1H), 1.79 - 1.69 (m, 1H), 1.26 - 1.16 (m, 6H).
**¹³C NMR** (100 MHz, DMSO-*d₆*) δ 173.3, 172.1, 172.0,171.2, 155.7, 144.9, 137.0, 128.5, 128.3, 127.8, 127.7, 127.4, 126.3, 69.3, 65.4, 51.6, 50.0, 47.9, 32.6, 27.3, 18.3, 18.1.
**HRMS (ESI)** m/z calcd. for C₃₈H₄₀N₄NaO₇ [M+Na]⁺: 687.2789, found: 687.2874.

### Example 14

A polypeptide of Formula 14 was prepared in the example. A specific process was as follows:

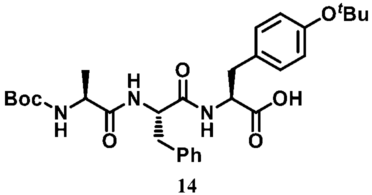

Boc-Ala-Phe-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the mixture was transferred to a system that was mixed with H-Tyr(O*^{t}*Bu)-OH (1 mmol), N,O-bistrimethylsilylacetamide (2 mmol) and 5 mL of dichloromethane in advance for reaction, and the reaction process was monitored by TLC. After the reaction was completed, 15 mL of water was added to acidify to pH=3 with 10% of citric acid. An aqueous phase was extracted once with ethyl acetate and washed twice with water, an organic phase was washed once with a saturated salt solution and dried with anhydrous sodium sulfate. The solvent was removed by distillation under reduced pressure, recrystallized with diethyl ether and petroleum ether, and the solid was filtered and collected to obtain a pure product, with a light yellow solid, *dr >* 99:1, and a yield of 91%.

Magnetic resonance imaging experimental data and mass spectrometry experimental data of products were as follows:
**¹H NMR** (400 MHz, DMSO-*d₆*) δ 8.35 - 8.22 (m, 1H), 7.80 - 7.66 (m, 1H), 7.27 - 7.11 (m, 7H), 6.94 - 6.78 (m, 3H), 4.64 - 4.52 (m, 1H), 4.48 - 4.37 (m, 1H), 3.99 - 3.82 (m, 1H), 3.07 - 2.96 (m, 2H), 2.93 - 2.84 (m, 1H), 2.82 - 2.69 (m, 1H), 1.36 (s, 9H), 1.26 (s, 9H), 1.06 (d, J = 6.6 Hz, 3H).
**¹³C NMR** (100 MHz, DMSO-*d₆*) δ 172.64, 172.15, 170.76, 154.86, 153.59, 137.44, 131.81, 129.53, 129.28, 127.86, 126.12, 123.34, 78.09, 77.58, 53.41, 53.20, 49.81, 37.67, 36.04, 28.53, 28.14, 18.14.
**HRMS (ESI)** m/z calcd. for C₃₀H₄₁N₃NaO₇ [M+Na]⁺: 578.2837, found: 578.2840.

### Example 15

A polypeptide of Formula 15 was prepared in the example. A specific process was as follows:

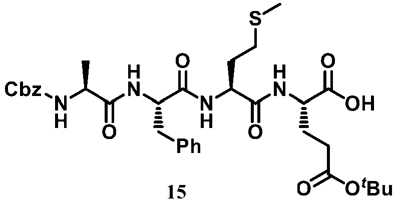

Cbz-Ala-Phe-Met-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the mixture was transferred to a system that was mixed with H-Glu(O*^{t}*Bu)-OH (1 mmol), hexamethyldisilylurea (1 mmol) and 3 mL of dichloromethane in advance for reaction, and the reaction process was monitored by TLC. After the reaction was completed, 15 mL of water was added to acidify to pH = 3 with 10% of citric acid. An aqueous phase was extracted twice with ethyl acetate, an organic phase was combined, washed once with water and washed once with a saturated salt solution and dried with anhydrous sodium sulfate. The solvent was removed by distillation under reduced pressure, recrystallized with dichloromethane and diethyl ether, and the solid was filtered and collected to obtain a pure product, with a white solid, *dr >* 99:1, and a yield of 92%.

Magnetic resonance imaging experimental data and mass spectrometry experimental data of products were as follows:
**¹H NMR** (400 MHz, DMSO-*d₆*) δ 8.13 (d, *J=* 7.5 Hz, 1H), 8.07 (d, *J =* 7.4 Hz, 1H), 7.93 (d, *J* = 7.3 Hz, 1H), 7.43 (d, *J=* 7.1 Hz, 1H), 7.40 - 7.28 (m, 5H), 7.25 - 7.15 (m, 5H), 5.09 - 4.93 (m, 2H), 4.60 - 4.49 (m, 1H), 4.44 - 4.36 (m, 1H), 4.28 - 4.20 (m, 1H), 4.05 - 3.98 (m, 1H), 3.09 - 3.00 (m, 1H), 2.90 - 2.80 (m, 1H), 2.49 - 2.40 (m, 2H), 2.33 - 2.23 (m, 2H), 2.03 (s, 3H), 2.02 - 1.89 (m, 2H), 1.88 - 1.74 (m, 2H), 1.39 (s, 9H), 1.14 (d, *J=* 7.0Hz, 3H).
**¹³C NMR** (100 MHz, DMSO-*d₆*) δ 173.0, 172.4, 171.5, 170.9, 170.7, 155.7, 137.6, 136.9, 129.3, 128.3, 127.9, 127.8, 127.7, 126.2, 79.8, 65.5, 53.6, 51.7, 51.1, 50.3, 37.2, 32.2, 31.2, 29.3, 27.7, 26.3, 18.0, 14.6.
**HRMS (ESI)** m/z calcd. for C₃₄H₄₇N₄O₉S [M+H]⁺: 687.3058, found: 687.3065.

### Example 16

A polypeptide of Formula 16 was prepared in the example. A specific process was as follows:

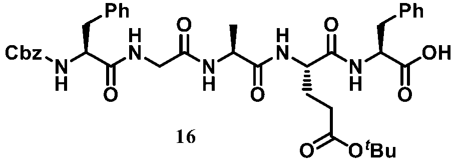

Cbz-Phe-Gly-Ala-Glu(tBu)-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the mixture was transferred to a system that mixed with H-Phe-OH (1 mmol), N-methyl-N-trimethylsilylacetamide (2 mmol) and 5 mL of acetonitrile in advance for reaction, and the reaction process was monitored by TLC. After the reaction was completed, 15 mL of water was added to acidify to pH=3 with 10% of citric acid. An aqueous phase was extracted twice with ethyl acetate, an organic phase was combined, washed once with water, washed once with a saturated salt solution and dried with anhydrous sodium sulfate. The solvent was removed by distillation under reduced pressure, recrystallized with dichloromethane and diethyl ether, and the solid was filtered and collected to obtain a pure product, with a white solid, *dr >* 99:1, and a yield of 90%.

Magnetic resonance imaging experimental data and mass spectrometry experimental data of products were as follows:
**¹H NMR** (400 MHz, DMSO-*d₆*) δ 8.43 - 8.25 (m, 1H), 8.12 - 7.89 (m, 3H), 7.57 (d, *J=* 7.1 Hz, 1H), 7.37 - 7.13 (m, 15H), 5.04 - 4.87 (m, 2H), 4.52 - 4.42 (m, 1H), 4.40 - 4.24 (m, 3H), 3.84 - 3.71 (m, 2H), 3.12 - 3.01 (m, 2H), 2.99 - 2.89 (m, 1H), 2.84 - 2.72 (m, 1H), 2.32 - 2.13 (m, 2H), 1.96 - 1.83 (m, 1H), 1.81 - 1.66 (m, 1H), 1.39 (s, 9H), 1.20 (d, *J =* 6.2 Hz, 3H).
**¹³C NMR** (100 MHz, DMSO-*d₆*) δ 172.7, 171.9, 171.9, 171.8, 170.9, 168.4, 156.0, 138.1, 137.3, 137.0, 129.2, 129.1, 128.3, 128.2, 128.0, 127.7, 127.4, 126.4, 126.2, 79.6, 65.3, 56.3, 53.4, 51.6, 48.1, 42.1, 37.3, 36.6, 31.2, 27.8, 27.5, 18.2.
**HRMS (ESI)** m/z calcd. for C₄₀H₅₀N₅O₁₀ [M+H]⁺: 760.3552, found: 760.3557.

### Example 17

A polypeptide of Formula 17 was prepared in the example. A specific process was as follows:

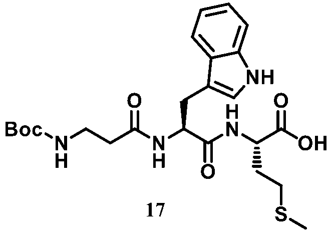

Boc-β-Ala-Trp-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that was mixed with H-Met-OH (1 mmol), N, O-bis(trimethylsilyl)trifluoroacetamide (1 mmol) and 3 mL of acetonitrile in advance for reaction, and the reaction process was monitored by TLC. After the reaction was completed, 15 mL of water was added to acidify to pH=3 with 10% of citric acid. An aqueous phase was extracted twice with ethyl acetate, an organic phase was combined, washed once with water, washed once with a saturated salt solution and dried with anhydrous sodium sulfate. The solvent was removed by distillation under reduced pressure, recrystallized with dichloromethane and diethyl ether, and the solid was filtered and collected to obtain a pure product, with a white solid, *dr >* 99:1, and a yield of 94%.

Magnetic resonance imaging experimental data and mass spectrometry experimental data of products were as follows:
**¹H** NMR (400 MHz, DMSO-*d₆*) δ 10.80 (s, 1H), 8.28 (d, *J =* 7.7 Hz, 1H), 8.06 (d, *J =* 8.1 Hz, 1H), 7.62 (d, *J =* 7.8 Hz, 1H), 7.33 (d, *J=* 8.0 Hz, 1H), 7.15 (s, 1H), 7.06 (t, *J =* 7.4 Hz, 1H), 6.98 (t, *J=* 7.4 Hz, 1H), 6.65 (t, *J =* 5.2 Hz, 1H), 4.66 - 4.53 (m, 1H), 4.41 - 4.29 (m, 1H), 3.18 - 3.01 (m, 3H), 2.96 - 2.85 (m, 1H), 2.52 - 2.41 (m, 2H), 2.29 - 2.14 (m, 2H), 2.04 (s, 3H), 2.02 - 1.95 (m, 1H), 1.95 - 1.84 (m, 1H), 1.36 (s, 9H).
**¹³C NMR** (100 MHz, DMSO-*d₆*) δ 173.2, 171.9, 170.3, 155.5, 136.1, 127.4, 123.6, 120.9, 118.5, 118.2, 111.3, 110.2, 77.6, 53.2, 51.0, 36.7, 35.7, 30.8, 29.7, 28.3, 27.7, 14.7.
**HRMS (ESI)** m/z calcd. for C₂₄H₃₅N₄O₆S [M+H]⁺: 507.2272, found: 507.2278.

### Example 18

A polypeptide of Formula 18 was prepared in the example. A specific process was as follows:

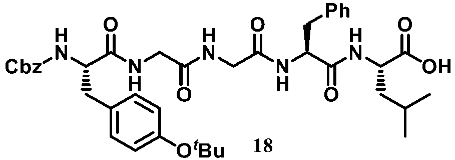

Cbz-Tyr(tBu)-Gly-Gly-Phe-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the dichloromethane was removed by distillation under reduced pressure and transferred to a system that mixed with H-Leu-OH (1 mmol), N,O-bistrimethylsilylacetamide (1 mmol) and 5 mL of N,N-dimethylformamide in advance for reaction, and the reaction process was monitored by TLC. After the reaction was completed, 15 mL of water was added to acidify to pH=3 with 10% of citric acid. An aqueous phase was extracted twice with ethyl acetate, an organic phase was combined, washed once with water, washed once with a saturated salt solution and dried with anhydrous sodium sulfate. The solvent was removed by distillation under reduced pressure, recrystallized with dichloromethane and diethyl ether, and the solid was filtered and collected to obtain a pure product, with a white solid, *dr >* 99:1, and a yield of 90%.

Magnetic resonance imaging experimental data and mass spectrometry experimental data of products were as follows:
**¹H NMR** (400 MHz, DMSO-*d₆*) δ 8.35 - 8.20 (m, 2H), 8.09 - 7.93 (m, 2H), 7.60 - 7.46 (m, 1H), 7.38 - 7.14 (m, 12H), 6.85 (d, *J =* 7.9 Hz, 2H), 5.04 - 4.84 (m, 2H), 4.70 - 4.54 (m, 1H), 4.36 - 4.20 (m, 2H), 3.82 - 3.70 (m, 3H), 3.69 - 3.62 (m, 1H), 3.12 - 2.97 (m, 2H), 2.84 - 2.65 (m, 2H), 1.74 - 1.49 (m, 3H), 1.26 (s, 9H), 0.97 - 0.80 (m, 6H).
**¹³C NMR** (100 MHz, DMSO-*d₆*) δ 173.9, 171.9, 171.1,168.9, 168.3, 155.9, 153.5, 137.8, 137.0, 132.7, 129.7, 129.3, 128.3, 128.0, 127.7, 127.5, 126.2, 123.3, 77.6, 65.3, 56.3, 53.6, 50.4, 42.1, 41.8, 40.0, 37.7, 36.8, 28.6, 24.3, 22.8, 21.4.
**HRMS (ESI)** m/z calcd. for C₄₀H₅₂N₅O₉ [M+H]⁺: 746.3760, found: 746.3766.

### Example 19

A polypeptide of Formula 19 was prepared in the example. A specific process was as follows:

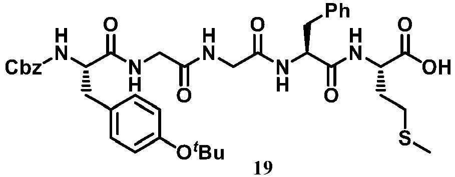

Cbz-Tyr(tBu)-Gly-Gly-OH (0.5 mmol), N-methyl-N-p-toluenesulfonyl ethynylamine (0.55 mmol) were taken to be dissolved in 5 mL of dichloromethane, a stirring reaction was performed at a room temperature, and the reaction process was monitored by TLC. When the raw acid was completely consumed, the mixture was transferred to a system that mixed with H-Phe-Met-OH (1 mmol), N-trimethylsilylpyrrolidone (1 mmol) and 5 mL of dichloroethane in advance for reaction, and the reaction process was monitored by TLC. After the reaction was completed, 15 mL of water was added to acidify to pH=3 with 10% of citric acid. An aqueous phase was extracted twice with ethyl acetate, an organic phase was combined, washed once with water, washed once with a saturated salt solution and dried with anhydrous sodium sulfate. The solvent was removed by distillation under reduced pressure, recrystallized with dichloromethane and diethyl ether, and the solid was filtered and collected to obtain a pure product, with a white solid, *dr >* 99:1, and a yield of 93%.

Magnetic resonance imaging experimental data and mass spectrometry experimental data of products were as follows:
**¹H NMR** (400 MHz, DMSO-*d₆*) δ 8.40 - 8.20 (m, 2H), 8.16 - 8.07 (m, 1H), 8.07 - 7.97 (m, 1H), 7.54 (d, *J =* 8.1 Hz, 1H), 7.35 - 7.15 (m, 12H), 6.86 (d, *J =* 7.6 Hz, 2H), 4.94 (s, 2H), 4.64 - 4.52 (m, 1H), 4.38 - 4.24 (m, 2H), 3.79 - 3.63 (m, 4H), 3.11 - 2.97 (m, 2H), 2.86 - 2.69 (m, 2H), 2.51 - 2.41 (m, 2H), 2.08 - 1.96 (m, 4H), 1.94 - 1.86 (m, 1H), 1.26 (s, 9H).
**¹³C NMR** (100 MHz, DMSO) δ 173.3, 172.0,171.1, 169.0,168.5, 156.0,153.5, 137.8, 137.0, 132.8, 129.7, 129.3, 128.3, 128.1, 127.7, 127.5, 126.3, 123.3, 77.6, 65.4, 56.3, 53.8, 51.3, 42.2, 41.9, 37.6, 36.8, 30.9, 29.7, 28.6, 14.6.
**HRMS (ESI)** m/z calcd. for C₃₉H₅₀N₅O₉S [M+H]⁺: 764.3324, found: 764.3330.

### Example 20

Carfilzomib was prepared in the example. A specific process was as follows:

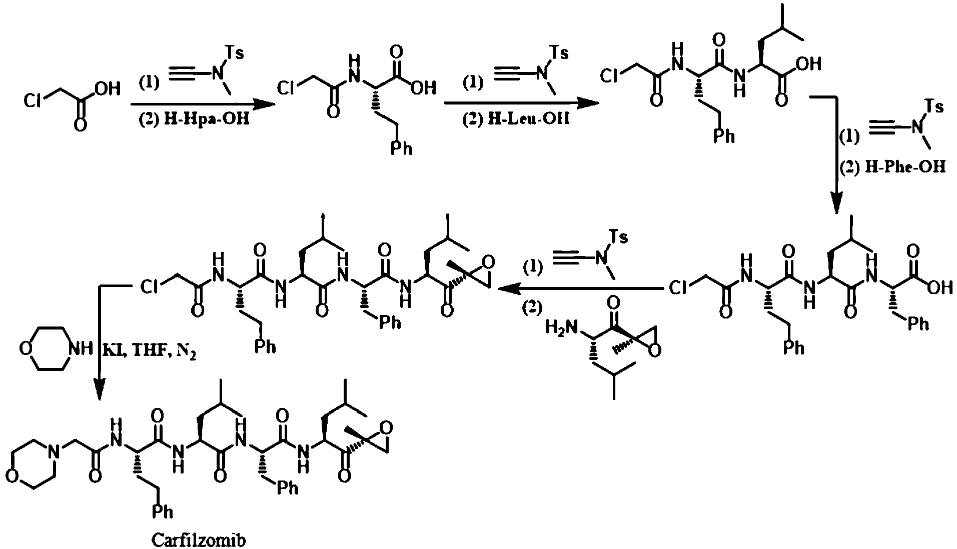

Chloroacetic acid (5 mmol) and N-methyl-N-p-toluenesulfonyl ethynylamine (5.5 mmol) were taken to be dissolved in 5 mL dichloromethane, a stirring reaction was performed at a room temperature, and the reaction process was monitored by TLC. When the raw acid was completely consumed, the mixture was transferred to a system that was mixed with L-homophenylalanine (10 mmol), N,O-bistrimethylsilylacetamide (10 mmol) and dichloromethane (5 mL) in advance for reaction, and the reaction process was monitored by TLC. After the reaction was completed, 20 mL of water was added to acidify to pH=2 with 1 M of hydrochloric acid. An aqueous phase was extracted twice with ethyl acetate, an organic phase was combined, washed once with water, washed once with a saturated salt solution and dried with anhydrous sodium sulfate. The solvent was removed by distillation under reduced pressure, recrystallized with dichloromethane and diethyl ether, and the solid was filtered and collected to obtain a pure product. The above steps were repeated to successively condense with L-phenylalanine, L-leucine and (2S)-2-amino-4-methyl-1-[(2R)-2-methyloxiranyl]-1-pentanone trifluoroacetate, the finally obtained intermediate was in a roomtemperature stirring reaction with morpholine (10 mmol), potassium iodide (KI, 5 mmol) and tetrahydrofuran (THF, 30 mL) in a reaction flask under nitrogen protection, and the reaction was monitored by TLC and High Performance Liquid Chromatography (HPLC).After the reaction was completed, the reaction solution was concentrated, water and ethyl acetate were added, the organic phase was extracted three times with ethyl acetate, combined and dried with anhydrous magnesium sulfate, the reaction solution was concentrated and recrystallized with ethyl acetate and petroleum ether to obtain the carfilzomib, with a white solid and a total yield of 65%. The following was the structural formula, magnetic resonance imaging experimental data and mass spectrometry experimental data of the product:
**¹H NMR** (400 MHz, DMSO-*d₆*) δ 8.25 (d, *J =* 6.8 Hz, 1H), 8.09 (d, *J* = 7.7 Hz, 1H), 7.97 (d, *J* = 7.7 Hz, 1H), 7.90 (d, *J =* 7.6 Hz, 1H), 7.33 - 7.23 (m, 2H), 7.22 - 7.02 (m, 8H), 4.61 - 4.49 (m, 1H), 4.42 - 4.33 (m, 2H), 4.32 - 4.23 (m, 1H), 3.71 - 3.50 (m, 4H), 3.41 - 3.32 (m, 1H), 3.12 (d, *J =* 4.2 Hz, 1H), 3.05 - 2.85 (m, 4H), 2.80 - 2.70 (m, 1H), 2.57 - 2.51 (m, 1H), 2.47 - 2.38 (m, 4H), 1.93 - 1.76 (m, 2H), 1.69 - 1.59 (m, 1H), 1.55 - 1.47 (m, 1H), 1.43 - 1.28 (m, 7H), 0.89 - 0.83 (m, 6H), 0.83 - 0.76 (m, 6H).
**¹³C NMR** (100 MHz, DMSO-*d₆*) δ 208.2, 171.5, 171.0, 170.9, 168.8, 141.4, 137.3, 129.1, 128.2, 128.2, 127.9, 126.1, 125.8, 66.0, 61.3, 58.7, 53.2, 53.0, 51.8, 51.5, 51.2, 49.2, 40.7, 38.5, 37.4, 34.3, 31.4, 24.5, 24.1, 23.2, 23.0, 21.6, 21.0, 16.3.
**HRMS** (ESI) m/z calcd. for C₄₀H₅₈N₅O₇ [M+H]⁺ : 720.4331, found: 720.4330.

### Example 21

Octreotide was prepared in the example. A specific process was as follows:

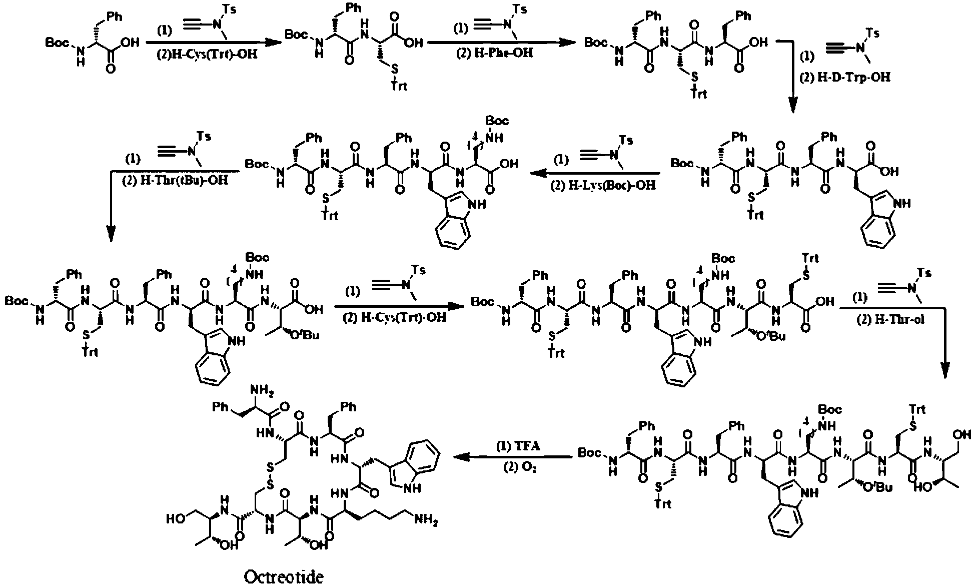

Boc-D-Phe-OH (5 mmol) and N-methyl-N-p-toluenesulfonyl ethynylamine (5.5 mmol) were taken to be dissolved in 20 mL dichloromethane, a stirring reaction was performed at a room temperature, and the reaction process was monitored by TLC. When the raw acid was completely consumed, the mixture was transferred to a system that was mixed with H-Cys(Trt)-OH (10 mmol), N,O-bistrimethylsilylacetamide (10 mmol) and dichloromethane (40 mL) in advance for reaction, and the reaction process was monitored by TLC. After the reaction was completed, 50 mL of water was added to acidify to pH = 3 with 10% of citric acid. An aqueous phase was extracted twice with ethyl acetate, an organic phase was combined, washed once with water, washed once with a saturated salt solution and dried with anhydrous sodium sulfate. The solvent was removed by distillation under reduced pressure, recrystallized with dichloromethane and diethyl ether, and the solid was filtered and collected to obtain Boc-D-Phe-Cys(Trt)-OH. The above steps were repeated to successively condense with H-D-Trp-OH, H-Lys(Boc)-OH, H-Thr(tBu)-OH, H-Cys(Trt)-OH and L-threoninol, to obtain the protected octreotide straight chain Boc-D-Phe-Cys(Trt)-Phe-D-Trp-Lys(Boc)-Thr(tBu)-Cys(Trt)-Thr-ol, the intermediate was added to a reaction flask that mixed with trifluoroacetic acid (42.5 mL), 1,2-ethanedithiol (2.5 mL), triisopropylsilane (2.5 mL) and water (2.5 mL) in advance, stirring was performed and the reaction was monitored by TLC and HPLC. After the reaction was completed, the reaction solution was concentrated, recrystallization was performed with diethyl ether to obtain the octreotide straight chain. Finally, the octreotide straight chain was dissolved in water, the concentration was diluted to 10⁻⁴ M, pH=8 was adjusted with 1 mmol of ammonium hydroxide, air was accessed at a room temperature for 48 h of oxidation, the octreotide was obtained after freeze-drying, with a white solid and a total yield of 56%. The following was the structural formula, magnetic resonance imaging experimental data and mass spectrometry experimental data of the product:
**¹H NMR** (400 MHz, DMSO-*d₆*) δ 10.88 (s, 1H), 9.04 (d, *J =* 8.2 Hz, 1H), 8.70 (d, *J =* 4.5 Hz, 1H), 8.52 (d, *J =* 7.5 Hz, 1H), 8.40 (d, *J =* 7.3 Hz, 1H), 8.34 (d, *J =* 8.4 Hz, 1H), 8.28 - 8.09 (m, 3H), 7.98 - 7.85 (m, 3H), 7.71 (d, *J =* 8.5 Hz, 1H), 7.45 (d, *J =* 7.8 Hz, 1H), 7.39 - 7.26 (m, 8H), 7.21 - 7.18 (m, 2H), 7.14 - 7.05 (m, 4H), 7.02 - 6.97 (m, 2H), 5.09 (d, *J* = 4.0 Hz, 1H), 5.03 - 4.97 (m, 1H), 4.90 - 4.84 (m, 1H), 4.83 - 4.72 (m, 1H), 4.64 (s, 1H), 4.42 (dd, *J =* 8.4, 5.4 Hz, 1H), 4.24 (dd, *J =* 14.5, 7.5 Hz, 2H), 4.12 - 4.06 (m, 1H), 4.00 - 3.92 (m, 2H), 3.66 - 3.61 (m, 1H), 3.18 (dd, *J =* 13.8, 5.2 Hz, 1H), 3.02 - 2.95 (m, 3H), 2.92 - 2.72 (m, 7H), 2.65 - 2.57 (m, 2H), 1.73 - 1.64 (m, 1H), 1.42 - 1.31 (m, 3H), 1.11 (d, *J =* 6.2 Hz, 3H), 1.05 (d, *J =* 6.4 Hz, 3H), 0.89 - 0.77 (m, 2H).
**¹³C NMR** (100 MHz, DMSO-*d₆*) δ 172.53, 171.87, 170.65, 170.39, 169.59, 168.47, 168.10, 136.81, 136.14, 134.76, 129.66, 129.04, 128.54, 128.09, 127.23, 127.05, 126.44, 123.69, 120.90, 118.26, 118.18, 111.34, 109.03, 66.63, 64.23, 60.20, 58.58, 55.90, 55.22, 53.97, 53.36, 53.23, 52.55, 52.01, 44.17, 42.39, 38.78, 38.52, 37.42, 30.26, 26.44, 26.38, 22.03, 19.92, 19.50.
**HRMS** (ESI) m/z calcd. for C₄₉H₆₇N₁₀O₁₀S₂ [M+H]⁺: 1019.4478, found: 1019.4484.

### Example 22

A protected lypressin straight chain was prepared in the example. A specific process was as follows:

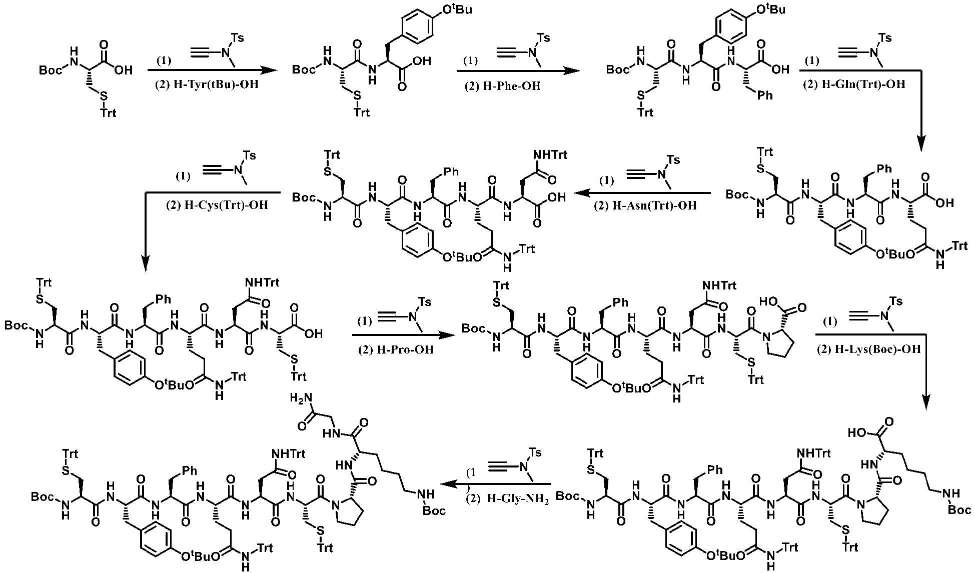

Boc-Cys(Trt)-OH (5 mmol) and N-methyl-N-p-toluenesulfonyl ethynylamine (5.5 mmol) were taken to be dissolved in 20 mL dichloromethane, a stirring reaction was performed at a room temperature, and the reaction process was monitored by TLC. When the raw acid was completely consumed, the mixture was transferred to a system that was mixed with H-Tyr(O*^{t}*Bu)-OH (10 mmol), N,O-bistrimethylsilylacetamide (10 mmol) and dichloromethane (40 mL) in advance for reaction, and the reaction process was monitored by TLC. After the reaction was completed, 50 mL of water was added to acidify to pH=3 with 10% of citric acid. An aqueous phase was extracted twice with ethyl acetate, an organic phase was combined, washed once with water, washed once with a saturated salt solution and dried with anhydrous sodium sulfate. The solvent was removed by distillation under reduced pressure, recrystallized with dichloromethane and diethyl ether, and the solid was filtered and collected to obtain Boc-Cys(Trt)-Tyr(O*^{t}*Bu)-OH. The above steps were repeated to successively condense with H-Phe-OH, H-Gln(Trt)-OH, H-Asn(Trt)-OH, H-Cys(Trt)-OH, H-Pro-OH, and H-Lys(Boc)-OH, H-Gly-NH₂ to obtain the protected lypressin straight chain Boc-Cys(Trt)-Tyr(O*^{t}*Bu)-Phe-Gln(Trt)-Asn(Trt)-Cys(Trt)-Pro-Lys(Boc)-Gly-NH₂, with a white solid and a total yield of 55%. The following was the structural formula, magnetic resonance imaging experimental data and mass spectrometry experimental data of the product:
**¹H NMR** (400 MHz, DMSO-*d₆*) δ 8.67 - 8.60 (m, 1H), 8.46 - 7.94 (m, 8H), 7.65 - 7.54 (m, 2H), 7.42 - 7.31 (m, 24H), 7.24 - 6.96 (m, 43H), 6.81 - 6.75 (m, 1H), 6.73 - 6.46 (m, 3H), 4.76 - 4.64 (m, 2H), 4.53 - 4.43 (m, 2H), 4.30 - 4.20 (m, 2H), 4.15 - 4.07 (m, 1H), 3.87 - 3.75 (m, 1H), 3.69 - 3.59 (m, 2H), 3.52 - 3.46 (m, 2H), 3.17 - 3.05 (m, 2H), 2.98 - 2.80 (m, 5H), 2.77 - 2.65 (m, 3H), 2.62 - 2.53 (m, 2H), 2.46 - 2.33 (m, 4H), 2.29 - 2.09 (m, 2H), 2.04 - 1.89 (m, 2H), 1.84 - 1.73 (m, 2H), 1.66 - 1.57 (m, 2H), 1.44 - 1.37 (m, 18H), 1.34 - 1.28 (m, 4H), 1.20 (s, 9H).
**¹³C NMR** (100 MHz, DMSO-*d₆*) δ 171.81, 171.77, 171.57, 171.34, 171.15, 170.85, 170.76, 170.66, 169.27, 168.60, 168.18, 155.57, 154.83, 153.24, 144.92, 144.71, 144.31, 144.26, 137.67, 132.03, 129.86, 129.84, 129.19, 129.11, 129.07, 128.58, 128.55, 128.07, 127.99, 127.46, 127.44, 126.70, 126.31, 126.14, 123.24, 78.53, 77.39, 69.42, 69.26, 66.45, 65.85, 59.31, 53.74, 53.14, 52.93, 52.01, 50.90, 49.79, 46.42, 41.98, 37.43, 37.33, 33.93, 32.72, 32.46, 31.30, 31.24, 29.30, 29.24, 29.17, 28.56, 28.32, 28.15, 24.25, 22.69, 22.63.
**HRMS** (ESI) m/z calcd. for C₁₃₆H₁₄₈N₁₃O₁₆S₂ [M+H]⁺: 2283.0603, found: 2283.0610.

The present disclosure discloses a method for synthesizing an amide and/or a polypeptide using a temporary protected amino acid as an amine component. In the present disclosure, the ynamide compound is used as the condensation agent, a low-cost unprotected amino acid is used as the raw material, and the silylation reagent is used to temporary protect the amino acid to perform the condensation reaction, so that "protection-condensation-deprotection" are carried out in one pot, and the three steps of reaction are combined into one step. After the construction of the peptide bond is achieved, the target polypeptide carboxylic acid can be obtained by a simple acid treatment, which can be directly used for the condensation of the next amino acid. At the same time, based on the characteristics of ynamide condensation agent, this method can also completely avoid the formation of racemic by-products, to ensure the yield and purity of the product. This method saves a large number of reagents and solvents used in the traditional methods of "protecting groups on amino acids" and "product deprotection", reduces the generation of a large amount of chemical waste, greatly reduces the cost of peptide synthesis, and improves the atom economy and step economy of peptide synthesis.

The above examples are preferred examples of the present disclosure, but the embodiments of the present disclosure are not limited to the above examples. Any other changes, modifications, substitutions, combinations and simplification made without departing from the spirit and principles of the present disclosure should be equivalent substitutions, and all included in the protection scope of the present disclosure.

## Claims

1. A method for preparing an amide and/or a polypeptide, **characterized by** comprising the following steps: 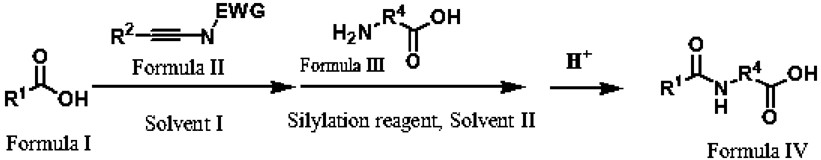
reacting a compound of formula I with a compound of formula II in a solvent I, and adding a compound of formula III, a silylation reagent and a solvent II to perform a nucleophilic substitution reaction, and then obtaining a compound of formula IV after acidification;
wherein R¹ is selected from alkyl, alkenyl, alkynyl, cycloalkyl, heterocycloalkyl, aryl, heteroaryl, an amino acid residual body lacking C-terminal carboxyl, and an amino acid derivative residual body or a polypeptide fragment lacking C-terminal carboxyl; R² is selected from hydrogen, deuterium, alkyl, cycloalkyl, heterocycloalkyl, aryl, heteroaryl, a silyl or an alkylsilyl; R³ is selected from alkyl, cycloalkyl, heterocycloalkyl, aryl and heteroaryl; R⁴ is selected from alkyl, cycloalkyl, heterocycloalkyl, aryl, heteroaryl, an amino acid residue or a polypeptide fragment; EWG is selected from nitro, cyano, sulfonyl, arylsulfonyl, alkanoyl and phosphonyl; the alkyl, alkenyl, alkynyl, cycloalkyl, heterocycloalkyl, aryl, heteroaryl, silyl and alkylsilyl are optionally unsubstituted, or substituted by one or more R¹¹; and each R¹¹ is independently selected from halogen, hydroxyl, carbonyl and C₁-C₆ alkyl.

2. The method for preparing the amide and/or the polypeptide according to claim 1, wherein when the solvent II is different from the solvent I, the method for preparing an amide and/or a polypeptide further comprises removing the solvent I after the reaction and then performing the nucleophilic substitution reaction.

3. A method for preparing an amide and/or a polypeptide, **characterized by** comprising the following steps: 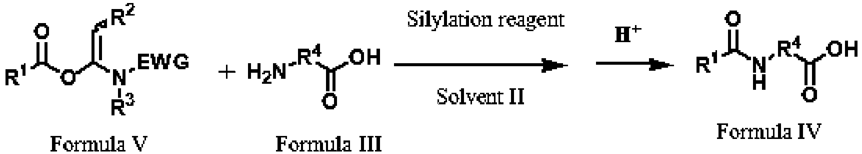
dissolving a compound of formula V, a compound of formula III and a silylation reagent in a solvent II to perform a nucleophilic substitution reaction, and then preparing a compound of formula IV after acidification;
wherein R¹ is selected from alkyl, alkenyl, alkynyl, cycloalkyl, heterocycloalkyl, aryl, heteroaryl, an amino acid residual body lacking C-terminal carboxyl, and an amino acid derivative residual body or a polypeptide fragment lacking C-terminal carboxyl; R² is selected from hydrogen, deuterium, alkyl, cycloalkyl, heterocycloalkyl, aryl, heteroaryl, a silyl or an alkylsilyl; R³ is selected from alkyl, cycloalkyl, heterocycloalkyl, aryl and heteroaryl; R⁴ is selected from alkyl, cycloalkyl, heterocycloalkyl, aryl, heteroaryl, an amino acid residue or a polypeptide fragment; EWG is selected from nitro, cyano, sulfonyl, arylsulfonyl, alkanoyl and phosphonyl; the alkyl, alkenyl, alkynyl, cycloalkyl, heterocycloalkyl, aryl, heteroaryl, silyl and alkylsilyl are optionally unsubstituted, or substituted by one or more R¹¹; and each R¹¹ is independently selected from halogen, hydroxyl, carbonyl and C₁-C₆ alkyl.

4. The method for preparing an amide and/or a polypeptide according to claim 1 or 3, wherein the silylation reagent comprises at least one of N-trimethylsilylacetamide, N-methyl-N-trimethylsilylacetamide, N-trimethylsilylpyrrolidone, N-(tert-butyldimethylsilyl)-N-methyltrifluoroacetamide, N,O-bis(tert-butyldimethylsilyl)acetamide, N,O-bistrimethylsilylacetamide, N, O-bis(trimethylsilyl)trifluoroacetamide, N-methyl-N-(trimethylsilyl)trifluoroacetamide, hexamethyldisilylurea, trimethylsilimidazole, dimethyl dichlorosilane, trimethylchlorosilane and tert-butyldimethylchlorosilane.

5. The method for preparing an amide and/or a polypeptide according to claim 1 or 3, wherein a molar ratio of the silylation reagent to the compound of formula III is (0.5-20): 1.

6. The method for preparing an amide and/or a polypeptide according to claim 1 or 3, wherein the solvent I comprises at least one of dichloromethane, chloroform, 1,2-dichloroethane, diethyl ether, toluene, acetonitrile, methanol or ethanol, and preferably the solvent II comprises at least one of acetonitrile, diethyl ether, ethylene glycol dimethyl ether, ethylene glycol diethyl ether, dioxane, dimethyl sulfoxide, N,N-dimethylformamide, N,N-dimethylacetamide, N-methylpyrrolidone, toluene, m-xylene, dichloromethane, 1,2-dichloroethane or chloroform.

7. The method for preparing an amide and/or a polypeptide according to claim 1 or 3, wherein the temperature of the nucleophilic substitution reaction is -20°C to 150°C.

8. The method for preparing an amide and/or a polypeptide according to claim 1 or 3, wherein the compound of formula III comprises any one of an amino acid compound, a polypeptide compound with free amino and carboxyl or other compounds with both amino and carboxyl.

9. The method for preparing an amide and/or a polypeptide according to claim 3, wherein the method for preparing an amide and/or a polypeptide comprises the following steps: dissolving the compound of formula III and the silylation reagent in a solvent II, adding the compound of formula V to perform the nucleophilic substitution reaction, and then preparing the compound of formula IV after acidification.

10. The method for preparing an amide and/or a polypeptide according to claim 3, wherein the method for preparing the compound of formula V comprises the following steps: 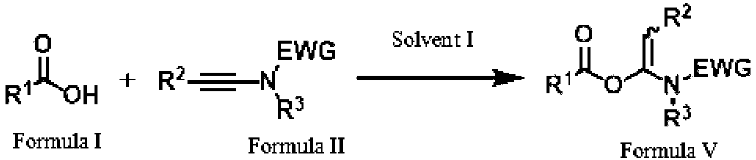
dissolving a compound of formula I and a compound of formula II in a solvent I to obtain a mixture, and reacting the mixture under stirring to prepare a compound of formula V;
wherein R¹, R², R³ and EWG are defined as in claim 1.

11. The method for preparing an amide and/or a polypeptide according to claim 1 or 3, wherein the method for preparing an amide and/or a polypeptide comprises the following steps: 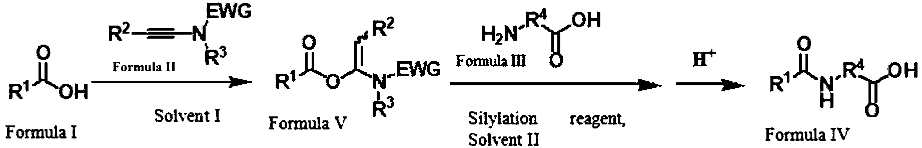
S1: dissolving the compound of formula I and the compound of formula II in the solvent I to obtain a mixture, and reacting the mixture under stirring to prepare a compound of formula V; and
S2: dissolving the compound of formula V, the compound of formula III and the silylation reagent in the solvent II to perform the nucleophilic substitution reaction, and then preparing a compound of formula IV after acidification;
wherein R¹, R², R³, R⁴ and EWG are defined as in claim 1 or 3.

12. The method for preparing an amide and/or a polypeptide according to claim 11, wherein the compound of formula I comprises at least one of fatty acid, aromatic acid, heterocyclic acid, acetylenic acid, olefine acid, amino acid, polypeptide carboxylic acid and derivatives of the above compounds.

13. The method for preparing an amide and/or a polypeptide according to claim 11, wherein a molar ratio of the compound of formula I to the compound of formula III is 1: (0.1-5).

14. Use of the method for preparing an amide and/or a polypeptide according to any one of claims 1 to 13 in preparing the amide and/or polypeptide.
